Europäisches Patentamt

European Patent Office

Office européen des brevets

(19)

(11) **EP 1 142 533 B1**

(12) **EUROPEAN PATENT SPECIFICATION**

(45) Date of publication and mention
of the grant of the patent:
**21.06.2006 Bulletin 2006/25**

(51) Int Cl.:
*A61B 5/11* *(2006.01)*

(21) Application number: **00830253.1**

(22) Date of filing: **04.04.2000**

(54) **"Monitoring and classification of the physical activity of a person"**

Beobachtung und Klassifizierung der Bewegungen einer Person

Surveillance et classification des activités physiques d'un sujet

(84) Designated Contracting States:
**DE FR GB IT**

(43) Date of publication of application:
**10.10.2001 Bulletin 2001/41**

(73) Proprietor: **STMicroelectronics S.r.l.**
**20041 Agrate Brianza (Milano) (IT)**

(72) Inventors:
• **Cuce, Antonino**
**26013 Crema (IT)**
• **Cassese, Maria**
**26013 Crema (IT)**
• **Platania, Davide**
**26013 Crema (IT)**

(74) Representative: **Pellegri, Alberto et al**
**c/o Società Italiana Brevetti S.p.A.**
**Piazza Repubblica, 5**
**21100 Varese (IT)**

(56) References cited:
**WO-A-86/04802**      **WO-A-93/16636**
**WO-A-99/04691**      **DE-A- 19 832 361**
**DE-C- 4 227 483**

**Description**

FIELD OF THE INVENTION

The present invention relates in general to data acquisition instruments of the motion activity of an individual in a totally instrumental manner as test data per se or to be correlated to simultaneous measurements of physiological parameters of the subject such as for example arterial pressure, breathing frequency, heart beat and alike.

BACKGROUND OF THE INVENTION

In medical field it is often useful to control patients while they go on their every day life. This permits to correlate parameters of clinical interest, such as for example arterial pressure, heart bit, etc. to the actual physical activity performed during daily activities. Such a monitoring and data acquisition is also extremely useful for medical research purposes because it allows to get more complete data as compared to those that may be obtained in an ambulatory under unnatural and/or constricting conditions.

Document WO-A-93 166 36 describes a roust-held monitoring device for physical condition.

Unfortunately it is not easy to implement means to effect measurements of such a kind, because if the patient is exerting himself physically, the recorded measuring may provide unreliable data because of the difficulty of correlating the various measurements to the current physical activity of the subject.

Typically the patient is recommended to note down on a diary the timing of daily activities performed, especially when the carried instrument actually takes a reading. However, besides aggravating with chores the monitored patient this practice may lead to voids and/or imprecisions in the recordings.

The ideal situation would be to combine to the instrument measuring the physiological parameters of clinical interest with a device capable of automatically detecting the physical activity being performed by the patient and to classify it for storing this information together with the readings periodically taken and recorded.

In this way, when the apparatus is given to the patient and properly installed to carry out correct measurements, the patient may indeed lead a normal life without worrying of the measurements been taken.

A solution of this type would be extremely advantageous because of a greatly enhanced reliability of the data. There would be a perfect synchronism between the detection and classification of the physical activity being performed and the measurement of the clinical parameter of interest. In this way the parameterization of data in function of the current state of the physical exertion would be extremely more coherent than that the patient who inadvertently could quantify in the same way different physical activities, could ever provide.

In simple words all the advantages that only an automatic data acquisition instrumentation can guarantee would be available.

It is evident the need and/or utility of an automatic system of detecting and storing parameters representative of the current state of physical exertion due to motion activity of the patient, during prolonged periods of monitoring in a totally instrumental manner without causing discomfort to the monitored subject.

OBJECT AND SUMMARY OF THE INVENTION

It has been found and is the object of the present invention an automatic system that can be carried (worn) by a person for acquiring and recording data on the movement of one or several parts of the body, detected by a corresponding number of sensors placed on respective parts of the body, the motion of which must be monitored, that fulfils in a optimal manner the above-noted needs and requisites.

Fundamentally, the automatic system of the invention is composed of

one or more motion sensors respectively applied to respective parts of the body of a subject been monitored;

an analog-digital converter associated to each sensor for converting the analog signal generated by the sensor into digital data;

a logic block of calculation of predefined parameters from successive time sequences of said digital data for each sensor;

first fuzzy logic means processing said calculated parameters and generating corresponding fuzzy logic labels of classification of the motion sensed by each of said sensors during the same interval of time of observation;

a storage memory of said parameters and/or fuzzy labels;

second fuzzy logic processing means of classification of the overall motion activity of the monitored subject fed with sets of fuzzy labels relative to the different motion sensors and outputting an index representing the physical activity been performed in the time interval of observation.

The use of fuzzy logic processing is itself extremely effective because the task is to assess hardly quantifiable (that is "fuzzy") quantities, the processing of which is greatly enhanced by using a fuzzy logic that is ideally structured for managing not sharply definable situations.

The invention and preferred embodiments thereof are clearly defined in the appended claims.

BRIEF DESCRIPTION OF DRAWINGS

**Figure 1** is a functional scheme of the system of classification of motion activity of the invention.
**Figure 2** is a functional diagram of a fuzzy logic labeler of the system of the invention.
**Figure 3** is a sample scheme of fuzzy logic classification of the motion activity according to an embodiment of the invention.
**Figure 4** is a scheme of fuzzy logic labeling relative to the considered example.
**Figure 5** is a fuzzy logic classification diagram of the motion activity.

DESCRIPTION OF SEVERAL EMBODIMENTS OF THE INVENTION

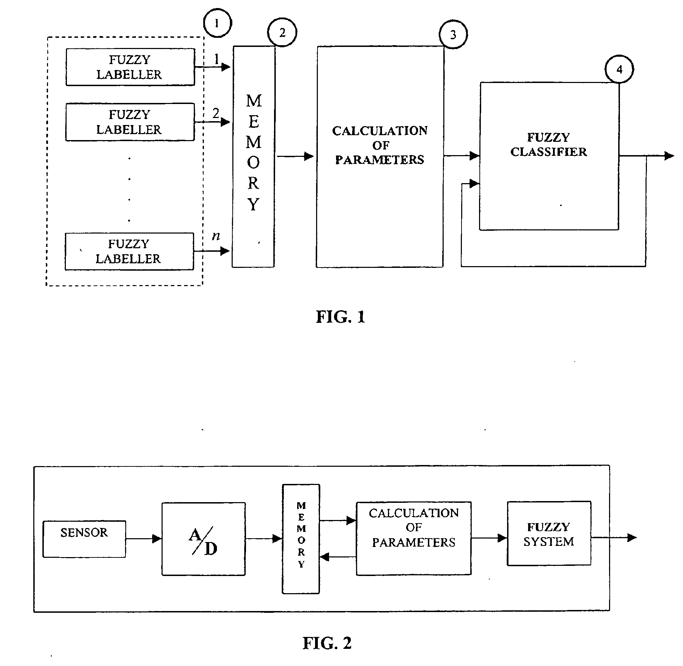

The system of motion activity classification based on fuzzy logic processing of the invention is schematically depicted in Figure 1.

The system of Figure 1 provides an overall classification of the motion activity of a subject by processing information on simultaneously detected movement of different parts of the body.

In block 1 of Figure 1 n fuzzy labelers are depicted. Each of them includes a sensor of motion and classifies the movement of a certain selected part of the body. Each fuzzy labeler dynamically outputs a sequence of "labels" that constitutes a classification of the movement detected in a certain time interval T.

For example, the following string may be output:

where labels "C", "N and "A" correspond respectively to a classification of the movement that has been performed in a fixed time interval T as: **calm, normal** and **restless.** Therefore in a time N*T, N labels are produced by each of the n labelers.

The monitoring is continuous and information on the motion been detected are stored in the memory 2.

From information thus obtained on the movement of different parts of the body, through a logic processing of the stored data performed by the calculation block 3, several numerical parameters are extracted.

For example, by fixing an interval of time t=N*T, in which as mentioned above, N labels of classification are detected for each of the n labelers, significative parameters that may be extracted are the numbers $N_C$, $N_N$ and $N_A$ corresponding to the number of times in which, among the N labels provided by anyone of the n labelers, a classification of movement, respectively as: **calm**, **normal** and **restless** is obtained.

The thus extracted parameters are then used for carrying out a classification of "second level" of the motion activity.

In practice, the object is that of using the information provided dynamically by the labelers on the "instantaneous" movement of the different parts of the body depending on the actual characteristics of the movement performed by the subject to be recognized, as well as of the parts of the body that are actually involved, in a procedure of classification. The following parameters are established:

• the interval of time t within which the calculation of the desired parameters must be effected;

• the significative parameters for the evaluation.

Finally, the fuzzy algorithm that is implemented in the block 4, is input with the so-extracted parameters and by applying a certain set of fuzzy rules classifies the overall motion activity of the subject during the time interval t by processing all the information (parameters) obtained on the movement of the different parts of the body of the monitored subject during the considered interval of time.

Both the movement classification of first level (block 1) and the classification of second level (block 4) take place dynamically and the system as a whole is able to monitor the actual motion activity of a subject over a long periods of time.

Taking into account that a fuzzy logic algorithm carries out a sort of "weighted average" of different information, it is important to underline that by performing a stepwise the classification of the movement besides enhancing reliability, makes the system more versatile. This because, in whichever way the overall motion is classified in the block 4 of overall classification, also the information on the instantaneous movement of the parts of the body is processed and stored.

### Fuzzy labeler

A detailed functional diagram of each single labeling device is depicted in Figure 2. Each device implements a first level classification of the movement.

The sensor, suitably chosen, among the many sensors of motion that can be satisfactorily used, detects the movement of the part of the body of interest and outputs an analog signal representative of the movement of the part of the body on which the sensor is applied. The analog signal is converted in digital data that are stored.

After every time interval T, that may be chosen by programming, the parameters that will be input to the fuzzy logic algorithm of global classification of the motion activity of the subject are calculated. The fuzzy logic processing block performs a classification of the movement of the particular part of the body as detected during each interval T.

### *Example*

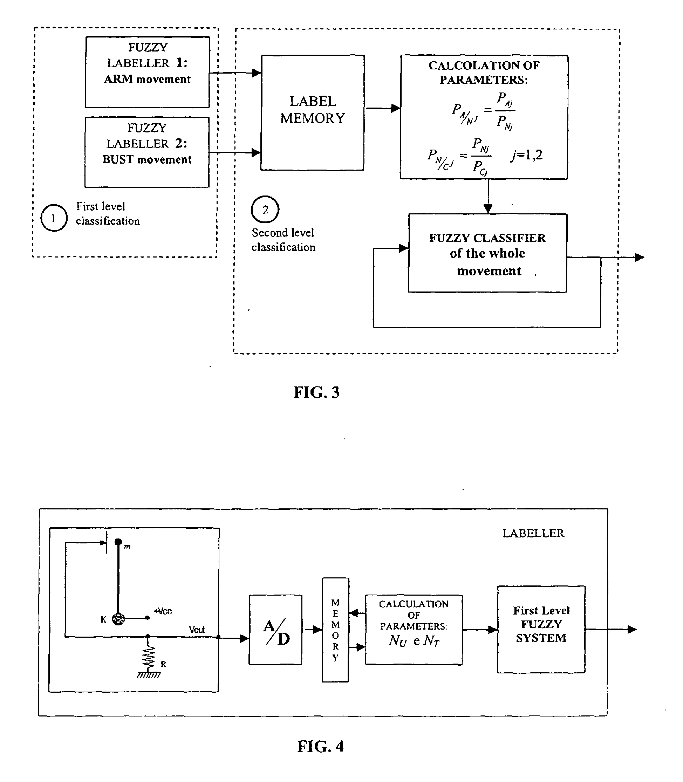

According to a sample embodiment that includes a first fuzzy labeler of the right arm movement and a second fuzzy labeler of the bust movement, the classifier of the overall motion activity of the invention is schematically depicted in Figure 3.

The movement classification is done in two steps:

• classification of the movement of the single parts of the body (arm and bust), or of first level. In this step two labelers are used, one for detecting and classifying the arm's movement and the other for detecting and classifying the bust movement.

• overall motion activity classification of second level, performed by using the information relative to the bust movement and arm movement produced in the first step.

### First level classification of the movement of the arm and of the bust

Classification of the movement of the arm and of the bust is performed by the block 1 of Figure 3, constituted by two identical fuzzy logic labelers, the diagram of which is depicted in Figure 4.

The sensor used is constituted by a cantilevered lamina restrained by a spring of elastic constant **K**. The metal lamina closes a circuit every time the system is subject to an acceleration such to overcome the force of the contrasting spring.

The output voltage Vout, assumes one or the other of two values according to whether the lamina closes or not the circuit. The analog signal provided by such an ON/OFF sensor is converted in a logic signal such that the bit "1" and "0" correspond to the ON and OFF condition of the contact of the circuit of the sensor.

Choosing for example a sampling frequency $f_c$=50 Hz, after every time interval T=5.1 sec., during which a byte is sampled and stored, the calculation of the parameters $N_U$ and $N_T$ according to the following equations is carried out:

$$N_U = \frac{\text{number of } 1}{f_c \cdot T} \tag{1}$$

$$N_T = \frac{\text{number of transitions } 0 \leftrightarrow 1}{f_c \cdot T} \tag{2}$$

It has been experimented and verified that there is a well defined relationship between the parameters $N_U$ and

$N_T$ and the type of corresponding movement. More in detail, the following results have been obtained.

- At rest or during calm conditions both parameters $N_U$ and $N_T$ are near to zero.

- The parameter $N_U$ provides a measure of the intensity of the movement. For example, it has been observed that by swinging the arm more and more vigorously, the values of $N_U$ proportionally increase.

- The parameter $N_T$ increases with the frequency of repeated abrupt movements.

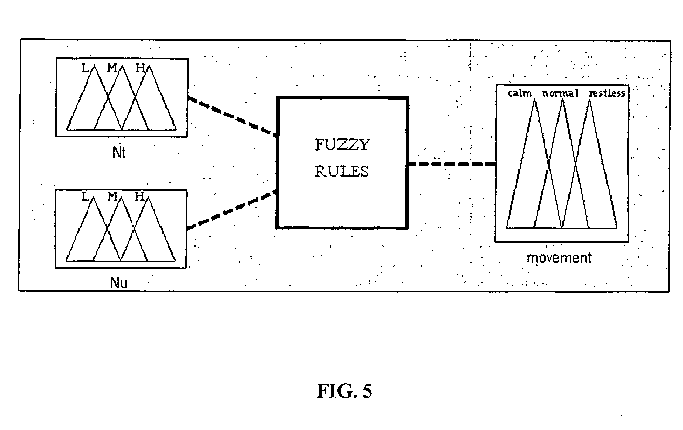

The experimental data have permitted to construct an efficient fuzzy algorithm of classification of the movement of the arm (and of the bust) based on the parameters $N_U$ and $N_T$.
The fuzzy logic processing scheme is illustrated in Figure 5.
On the base of the experimental measurements, the value that the parameters $N_U$ and $N_T$ may assume have been grouped in three fuzzy sets corresponding to the indexes: **Low, Medium** and **High.** Similarly, the arm movement (and the bust movement) has been divided in three fuzzy classes, corresponding to: **calm (C)**, **normal (N)** and **restless (A).**
The fuzzy rules have been defined by contemplating all possible cases. In practice, in all the rules, the antecedent contains both input parameters and are contemplated all possible combinations among the fuzzy sets of both parameters. In total, having each variable 3 membership functions, the block of fuzzy rules contains $3^2=9$ rules of the type:
**IF** $N_T$ **IS** *Low* **AND** $N_U$ **IS** *Low* **THAN** *movement* **IS** *Calm*
**IF** $N_T$ **IS** *Medium* **AND** $N_U$ **IS** *Low* **THAN** *movement* **IS** *Calm*
**IF** $N_T$ **IS** *Low* **AND** $N_U$ **IS** *Medium* **THAN** *movement* **IS** *Normal*
etc.
etc.
It has been verified that the labeling system thus implemented provides a reliable classification of the movement of even other parts of the body. Of course, by adding other sensors and relative labeling systems, it is possible to generate direct information on the movement of other parts of the body for refining if necessary the monitoring and the precision of the overall assessment of the motion activity of the subject.

Classification of second level of the overall motion activity

The classification of the overall motion activity of a subject is implemented by the block 2 of Figure 3, by assembling information on the right arm movement, obtained by the two respective identical labeling systems.
After each time interval n*T, where T is the time necessary to receive a classification label, each labeling system outputs a string of the type:

LABELER 1: arm

| A | A | A | N | N | N | C | N | C | C | C |

LABELER 2: bust

| N | N | N | N | C | N | N | C | C | C | C |

0  T  2T  3T  4T ····  n·T

In the example, the interval of time n*T within which the classification is performed has been chosen equal to 1 minute: n*T= 12*5.1 sec $\cong$ 1 min
From each string of n labels the following parameters are extracted:

$$P_{Cj}, P_{Nj} \text{ e } P_{Aj} \qquad j=1,2$$

They represent the number of presences, in a string, of labels relative to the calm (C), normal (N) and restless (A) classification, respectively. A different weight is assigned to each count depending on the position of the relative label in the string. The greater weight is for labels that concentrate in the final portion of the string been processed.
The chosen input parameters of the fuzzy logic algorithm of second level classification are:

$$P_{A/N^j} = \frac{P_{A_j}}{P_{N_j}} \qquad\qquad (3)$$

$$P_{N/C^j} = \frac{P_{N_j}}{P_{C_j}} \qquad\qquad j = 1,2 \qquad\qquad (4)$$

The fuzzy logic algorithm of second level classification has been developed on the base of experimental observations and in particular the fuzzy sets and the block of rules. In developing the rules, it has been accounted for the fact that different parts of the body contribute in a different way toward an overall assessment of the motion activity of the subject been monitored. For example, should, according to a first level classification, be present a conspicuously rhythmic movement of the arm and simultaneously a calm condition of the bust, the subject would be classified as being in a semi-calm overall condition. Therefore, the fuzzy sets of second level are structured such to weight differently the classification (labeling) of the movement of different parts of the body.

The values that each parameter may assume are grouped in three fuzzy sets corresponding to **Low**, **Medium** and **High**. The memberships Low, Medium and High are different for each input parameter. In particular the thresholds that fix the passage from a fuzzy set to another are suitably shifted depending on whether the input parameter refers to the arm or to the bust.

Depending on the values of the input parameters, the block of fuzzy rules classifies the overall motion activity, as calm, normal or restless, according to the scheme depicted in Figure 6.

In this step of second level classification, a larger number of parameters must be processed compared to the first level classification. Each fuzzy rule is structured such that the antecedent contains information on both the labelers of the considered example.

The rules are of the type:

**IF** $P_{A/N}1$ **IS** *Low* **AND** $P_{A/N}2$ **IS** *Low* **THAN** *movement* **IS** *Calm*
**IF** $P_{A/N}1$ **IS** *Medium* **AND** $P_{A/N}2$ **IS** *Low* **THAN** *movement* **IS** *Calm*
**IF** $P_{A/N}1$ **IS** *Low* **AND** $P_{A/N}2$ **IS** *Medium* **THAN** *movement* **IS** *Normal*
etc.
etc.

As already said above, the system functions in a dynamic way. Therefore, instant by instant the system produces a classification of the overall motion activity carried out for the n*T preceding seconds.

The prototype system that has been developed has proven itself outstandingly suited to classify the overall motion activity of a subject, providing information useful toward a precise characterization of the current state of physical exertion of a patient. The system has been used as a support for a pressure holter and the additional information on the motion activity at the time of the taking of each pressure reading reveals itself extremely useful for more reliably analyzing the collected blood pressure data provided by the holter.

Its principle of operation makes the system of the invention extremely versatile and readily adaptable to different needs.

The block of fuzzy rules may be easily reprogrammed, allowing exploitation of additional information on the movement of other parts of the body.

In particular, it is possible to obtain instantaneous information on different parts of the body, by elaborating which an overall characterization of the physical activity performed in more or less long periods of time may be obtained, to satisfy specific requisites.

## Claims

1.  A portable automatic system of acquisition and recording of information on the movement of one or more parts of the body of a monitored subject through a corresponding number of motion sensors placed on respective parts of the body, the movement of which must be detected and of assessment data on the global motion activity of the subject during an established interval of time, comprising
one or more motion sensors respectively placed on parts of the body of a subject under observation;
an analog-to-digital converter associated to each sensor converting an analog signal generated by the sensor into digital data;
a logic block of calculation of predefined parameters from successive sequences of said digital data for each sensor;
first fuzzy logic means processing said calculated parameters and generating corresponding fuzzy labels of classification of the movement detected by each of said sensors during the same established interval of time;
a storage memory of said parameters and/or fuzzy labels; and **characterized in that** there is a second fuzzy logic processing means of classification of the global motion activity of the subject under observation input with sets of fuzzy labels relative to the different motion sensors and outputting a representative index of the global motion activity of the subject in said interval.

2. The system according to claim 1, wherein said memory supports also the storage of said sequences of digital data produced by each sensor.

3. The system according to claim 1, **characterized by** comprising at least two motion sensors, a first sensor been applied to an arm and a second sensor been applied to the bust of the subject under observation.

4. The system according to claim 1, wherein said sensors detect the presence or absence of acceleration in an ON/OFF mode.

5. The system according to claim 4, wherein said calculated parameters for each sensor represent the total time of persistence of acceleration during a fixed interval of time of monitoring and the number of ON/OFF and OFF/ON transitions occurred during said interval of observation.

6. The system according to claim 1, wherein said fuzzy labels of classification of the movement detected by each of said sensors during each interval of time are three, qualifying the movement as either calm, normal or restless.

**Patentansprüche**

1. Tragbares automatisches System zur Erfassung und Aufzeichnung von Informationen über die Bewegung eines oder mehrerer Teile des Körpers eines überwachten Probanden durch eine entsprechende Anzahl von Bewegungssensoren, die an jeweiligen Teilen des Körpers angeordnet sind, dessen Bewegung detektiert werden muß, und zur Bewertung von Daten über die allgemeine Bewegungsaktivität des Probanden während eines festgelegten Zeitintervalls, das aufweist:

einen oder mehrere Bewegungssensoren, die jeweils an Teilen des Körpers eines beobachteten Probanden angeordnet sind;
einen Analog-Digital-Wandler, der mit jedem Sensor verbunden ist und ein durch den Sensor erzeugtes Analogsignal in digitale Daten umwandelt;
einen Logikblock zur Berechnung vordefinierter Parameter aus aufeinanderfolgenden Sequenzen der digitalen Daten für jeden Sensor;
eine erste Fuzzy-Logikeinrichtung, die die berechneten Parameter verarbeitet und entsprechende Fuzzy-Kennungen der Klassifizierung der Bewegung erzeugt, die durch jeden der Sensoren während desselben festgelegten Zeitintervalls detektiert werden;
einen Speicher für die Parameter und/oder Fuzzy-Kennungen; und das **dadurch gekennzeichnet ist, daß** es eine zweite Fuzzy-Logikverarbeitungseinrichtung der Klassifizierung der allgemeinen Bewegungsaktivität des beobachteten Probanden gibt, in die Sätze von Fuzzy-Kennungen bezüglich der unterschiedlichen Bewegungssensoren eingegeben werden, und die einen repräsentativen Index der allgemeinen Bewegungsaktivität des Probanden in dem Intervall ausgibt.

2. System nach Anspruch 1, wobei der Speicher außerdem die Speicherung der Sequenzen digitaler Daten unterstützt, die durch jeden Sensor erzeugt werden.

3. System nach Anspruch 1, **dadurch gekennzeichnet, dass** es mindestens zwei Bewegungssensoren aufweist: einen ersten Sensor, der an einen Arm angelegt worden ist, und einen zweiten Sensor, der an die Brust des beobachteten Probanden angelegt worden ist.

4. System nach Anspruch 1, wobei die Sensoren das Vorhandensein oder Fehlen einer Beschleunigung in einer EIN/AUS-Betriebsart detektieren.

**5.** System nach Anspruch 4, wobei die berechneten Parameter für jeden Sensor die Gesamtzeit der Fortdauer der Beschleunigung während eines festen Zeitintervalls der Überwachung und die Anzahl der EIN/AUS- und AUS/EIN-Übergänge repräsentieren, die während des Beobachtungsintervalls auftreten.

**6.** System nach Anspruch 1, wobei die Fuzzy-Kennungen der Klassifizierung der Bewegung, die durch jeden der Sensoren während jedes Zeitintervalls detektiert werden, drei sind, die die Bewegung entweder als ruhig, normal oder ruhelos qualifizieren.

**Revendications**

**1.** Système automatique portable d'acquisition et d'enregistrement d'informations sur le déplacement d'une ou plusieurs parties du corps d'un sujet surveillé par l'intermédiaire d'un nombre correspondant de capteurs de mouvement placés sur des parties respectives du corps dont le mouvement doit être détecté, et de déduction de données sur l'activité de mouvement globale du sujet pendant un intervalle de temps donné, comprenant :

un ou plusieurs capteurs de mouvement placés respectivement sur les parties du corps d'un sujet sous observation ;
un convertisseur analogique-numérique associé à chaque capteur, convertissant un signal analogique produit par le capteur en données numériques ;
un bloc logique de calcul de paramètres prédéfinis à partir de séquences successives desdites données numériques pour chaque capteur;
de premiers moyens de logique floue traitant les paramètres calculés et produisant des étiquettes floues correspondantes de classification du mouvement détecté par chacun des capteurs pendant le même intervalle de temps donné ;
une mémoire de stockage des paramètres et ou des étiquettes floues ; et
**caractérisé en ce qu'**il est prévu un second moyen de traitement en logique floue de classification de l'activité de mouvement globale du sujet sous observation recevant des ensembles d'étiquettes floues relatives aux différents capteurs de mouvement et fournissant un indice représentatif de l'activité de mouvement globale du sujet dans ledit intervalle.

**2.** Système selon la revendication 1, dans lequel la mémoire permet également le stockage des séquences de données numériques produites par chaque capteur.

**3.** Système selon la revendication 1, **caractérisé en ce qu'**il comprend au moins deux capteurs de mouvement, un premier capteur étant appliqué à un bras et un second capteur étant appliqué au buste du sujet sous observation.

**4.** Système selon la revendication 1, dans lequel les capteurs détectent la présence ou l'absence d'accélération en tout ou rien.

**5.** Système selon la revendication 4, dans lequel les paramètres calculés pour chaque capteur représentent la durée totale de persistance d'accélération pendant un intervalle de temps fixe de surveillance et le nombre de transitions marche/arrêt et arrêt/marche qui surviennent pendant ledit intervalle de temps d'observation.

**6.** Système selon la revendication 1, dans lequel les étiquettes floues de classification du mouvement détecté par chacun des détecteurs pendant chaque intervalle de temps sont au nombre de trois, qualifiant le mouvement comme calme, normal ou agité.

FIG. 1

FIG. 2

FUZZY
LABELLER 1:
ARM movement

FUZZY
LABELLER 2:
BUST movement

First level
classification
①

LABEL
MEMORY

Second level
classification
②

CALCOLATION OF
PARAMETERS:

$$P_{A/N^j} = \frac{P_{Aj}}{P_{Nj}}$$

$$P_{N/C^j} = \frac{P_{Nj}}{P_{Cj}} \qquad j=1,2$$

FUZZY CLASSIFIER
of the whole
movement .

**FIG. 3**

LABELLER

m

K          +Vcc

Vout

R

$\frac{A}{D}$

MEMORY

CALCULATION
OF
PARAMETERS:
$N_U$ e $N_T$

First Level
FUZZY
SYSTEM

**FIG. 4**

**FIG. 5**